(19) Europäisches Patentamt
European Patent Office
Office européen des brevets

(11) Veröffentlichungsnummer: **0 027 189**
**B1**

# (12) EUROPÄISCHE PATENTSCHRIFT

(45) Veröffentlichungstag der Patentschrift: **22.02.84**

(51) Int. Cl.[3]: **C 07 D 487/14**, C 07 D 249/10 // (C07D487/14, 249/00, 249/00, 241/00)

(21) Anmeldenummer: **80105718.3**

(22) Anmeldetag: **24.09.80**

(54) **Derivat des 2.5-Diketopiperazines, Verfahren zu seiner Herstellung und seine Verwendung.**

(30) Priorität: **06.10.79 DE 2940654**

(43) Veröffentlichungstag der Anmeldung: **22.04.81 Patentblatt 81/16**

(45) Bekanntmachung des Hinweises auf die Patenterteilung: **22.02.84 Patentblatt 84/8**

(84) Benannte Vertragsstaaten: **BE CH DE FR GB LI NL**

(56) Entgegenhaltungen:
**DE - A - 2 411 823**

**Chem. Abs., 84(1976), 105479a(=Nippon Kagaku Kaishi 1975, 12, S. 2244-45)**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Beck, Gunther, Dr., Am Mittelberg 19, D-5090 Leverkusen 1 (DE)**

**EP 0 027 189 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Derivat des 2,5-Diketopiperazins, Verfahren zu seiner Herstellung und seine Verwendung

Die vorliegende Erfindung betrifft ein neues Derivat des 2,5-Diketopiperazins, ein Verfahren zu seiner Herstellung und seine Verwendung. 2,5-Diketopiperazine, wie beispielsweise Diimidazo[1,5-a,1',5'-d]-pyrazin-4,9-dion, sind aus Nippon Kagaku Kaishi, 1975, 12, S. 2244–2245 (Chem. Abs. 84 [1976], 105479a), bekannt. Die Herstellung erfolgt aus Imidazol-4-carbonsäure mit $PCl_5/SOCl_2$ ohne Zusatz von Dimethylformamid.

Das neue Derivat des 2,5-Diketopiperazins entspricht der Formel

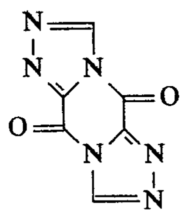

(I)

Es wurde auch ein Verfahren zur Herstellung des Derivats des 2,5-Diketopiperazins gefunden, das dadurch gekennzeichnet ist, daß man die 1,2,4-Triazol-3-carbonsäure mit einer mindestens äquimolaren Menge eines Säurechlorids oder -bromids von Säuren des Schwefels, des Phosphors oder des Kohlenstoffes in Gegenwart von 0,5 bis 20 Mol-% Dimethylformamid, gegebenenfalls in Gegenwart eines inerten Lösungs- bzw. Verdünnungsmittels, bei erhöhter Temperatur umsetzt.

Für das erfindungsgemäße Verfahren werden die Säurechloride und die Säurebromide, bevorzugt die Säurechloride, von Säuren des Schwefels, des Phosphors und des Kohlenstoffes eingesetzt, wie Thionylchlorid, Thionylbromid, Phosphortrichlorid, Phosphortribromid, Phosphorpentachlorid, Phosphorpentabromid, Phosphoroxichlorid, Phosphoroxibromid, Oxalylchlorid und Phosgen.

Zur Erzielung eines vollständigen Umsatzes ist im erfindungsgemäßen Verfahren eine mindestens äquimolare Menge des Säurechlorids oder -bromids, bezogen auf die 1,2,4-Triazol-3-carbonsäure, erforderlich. Es kann jedoch auch eine größere Menge Säurechlorid oder -bromid, bezogen auf die Triazol-carbonsäure, beispielsweise im Molverhältnis bis zu 10 : 1, eingesetzt werden. Falls überschüssiges Säurechlorid oder -bromid eingesetzt wird, kann dieses nach Beendigung der Reaktion zurückgewonnen und in einem folgenden Ansatz wieder eingesetzt werden.

Die Umsetzung nach dem erfindungsgemäßen Verfahren wird in Gegenwart von 0,5 mit 20 Mol-%, bevorzugt von 1 bis 10 Mol-%, Dimethylformamid, bezogen auf die eingesetzte Triazolcarbonsäure, durchgeführt.

Das erfindungsgemäße Verfahren kann grundsätzlich mit oder ohne Lösungs- bzw. Verdünnungsmittel durchgeführt werden. Die Variante ohne Lösungs- bzw. Verdünnungsmittel ist beispielsweise dann möglich, wenn das benutzte Säurechlorid oder -bromid bei der gewählten Reaktionstemperatur flüssig ist und insbesondere dann, wenn es in einem Überschuß — wie oben beschrieben — eingesetzt wird.

Für den Fall einer Durchführung des erfindungsgemäßen Verfahrens in Gegenwart eines Lösungs- bzw. Verdünnungsmittels seien beispielsweise genannt: Kohlenwasserstoffe, wie Benzol, Toluol, Xylol, Cyclohexan oder Petrolether; chlorierte Kohlenwasserstoffe, wie Chloroform, Tetrachlorkohlenstoff, Tetrachlorethylen, 1,1,2,2-Tetrachlorethan oder Chlorbenzol; Ether, wie 1,4-Dioxan oder Tetrahydrofuran.

Die bevorzugte Variante ist die, daß ein bei der Reaktionstemperatur flüssiges Säurechlorid oder -bromid gleichzeitig als Lösungs- bzw. Verdünnungsmittel dient und in diesem Fall in einer Menge von 2 bis 10 Mol Säurechlorid oder -bromid pro Mol Triazolcarbonsäure eingesetzt wird.

Als erhöhte Temperatur für das erfindungsgemäße Verfahren sei beispielsweise eine Temperatur von 40 bis 150°C, bevorzugt von 50 bis 100°C, genannt. In besonders bevorzugter Weise wird beim Siedepunkt des eingesetzten Säurechlorids oder -bromids gearbeitet.

Das erfindungsgemäße Verfahren kann bei Normaldruck, Überdruck oder Unterdruck durchgeführt werden. Die bevorzugte Variante ist die Durchführung bei Normaldruck. Jedoch kann beispielsweise auch ein Unterdruck bei einem hochsiedenden Säurechlorid oder -bromid oder ein Überdruck bei einem niedrigsiedenden Säurechlorid oder -bromid angewandt werden, um die gewünschte Reaktionstemperatur beim Siedepunkt des jeweiligen Säurechlorids oder -bromids einzustellen. Als Überdruck sei in einem solchen Fall beispielsweise der Eigendruck der Reaktionsmischung bei der gewählten Reaktionstemperatur genannt.

Die 1,2,4-Triazol-3-carbonsäure ist bekannt und kann beispielsweise durch Oxidation von 3-Methyl-1,2,4-triazol mit alkalischer Permanganatlösung (Atti della Reale Accadamia dei Lincei Roma, Serie 4, Band 7 II, Seite 462; zitiert nach Beilsteins Handbuch der Organischen Chemie, 4. Auflage, Band 26, Seite 280, Verlag Julius Springer, Berlin, 1937) oder durch Reduktion von diazotierter 5-Amino-1,2,4-Triazol-3-carbonsäure mit Methanol (Khimiya Geterotsiklicheskikh Soedinenii, Vol. 1 [1965], No. 4, Seite 420 der englischen Übersetzung) gewonnen werden.

Das erfindungsgemäße Verfahren sei unter Benutzung von Thionylchlorid als Säurechlorid durch die folgende Reaktionsgleichung erläutert:

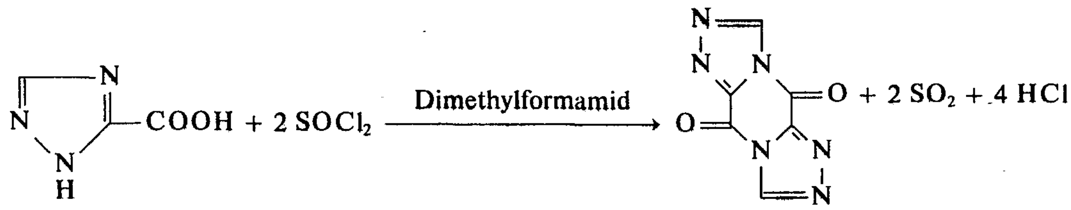


Zur Durchführung des erfindungsgemäßen Verfahrens wird die 1,2,4-Triazol-3-carbonsäure gemeinsam mit dem Säurechlorid oder -bromid, dem Dimethylformamid sowie gegebenenfalls dem Lösungs- bzw. Verdünnungsmittel unter Rühren auf die gewählte Reaktionstemperatur erhitzt.

Nach Beendigung der Gasentwicklung wird das Derivat des 2,5-Diketopiperazins im allgemeinen durch Filtration aus dem Reaktionsgemisch abgetrennt und liegt dann bereits in reiner Form vor. Es ist jedoch auch möglich, alle flüchtigen Bestandteile des Reaktionsgemisches durch Destillation, gegebenenfalls im Vakuum, abzutrennen. Falls es erforderlich erscheint, kann das Derivat des 2,5-Diketopiperazins durch übliche Reinigungsmethoden, beispielsweise durch Sublimation, Vakuum-Sublimation oder Umkristallisation weiter gereinigt werden.

Das neue Derivat des 2,5-Diketopiperazins stellt eine wertvolle Ausgangsverbindung für organische Synthesen, beispielsweise auf pharmazeutischem Gebiet, dar. So läßt sich beispielsweise aus dem Derivat des 2,5-Diketopiperazins durch Erwärmen mit wäßrigem Ammoniak das 1,2,4-Triazol-3-carbonsäureamid herstellen. Dieses kann als Antivirus-Mittel (belgisches Patent 812 191) sowie als Medikament zur Behandlung von Arthritis und Rheumatismus (deutsche Offenlegungsschriften 2 533 926 und 2 535 428) verwendet werden. Das 1,2,4-Triazol-3-carbonsäureamid kann weiterhin mit dem Enzym Nucleosid-Phosphorylase in Gegenwart von Ribose-1-phosphat zu 1-$\beta$-D-ribofuranosyl-1,2,4-triazol-3-carbonsäureamid umgesetzt werden (DDR-Patentanmeldung 114 413), welches als Antivirus-Mittel bei Erkrankungen der Atemwege und als Medikament zur Behandlung der Arthritis und des Rheumatismus (deutsche Offenlegungsschrift 2 535 428) eingesetzt werden.

## Beispiel 1

Eine Suspension von 113 g (1 Mol) 1,2,4-Triazol-3-carbonsäure in 500 ml (6,9 Mol) Thionylchlorid wird mit 5 ml (etwa 0,07 Mol) Dimethylformamid versetzt und anschließend unter Rühren 2 Stunden unter Rückfluß zum Sieden erhitzt. Nach dem Abkühlen auf Raumtemperatur wird abgesaugt, mit etwas Thionylchlorid nachgewaschen und unter Feuchtigkeitsausschluß getrocknet. Man erhält 90 g (entsprechend 95% der theoretischen Ausbeute) an dem Derivat des 2,5-Diketopiperazins.

Die Verbindung ist bei 200° C/0,01 Torr in farblosen, sechseckigen Kristallen sublimierbar und ist bei 290° C noch nicht geschmolzen. IR-Spektrum (KBr-Preßling, in $cm^{-1}$): 3105, 1858, 1772, 1540, 1425, 1345, 1284, 1147, 1004, 750, 699, 636.

Massenspektrum: Berechnet für $C_6H_2N_6O_2$ 190, gefunden 190.

## Beispiel 2

### Herstellung von 1,2,4-Triazol-3-carbonsäureamid aus dem Derivat des 2,5-Diketopiperazins

Das Derivat des 2,5-Diketopiperazins wird in etwa der fünffachen Gewichtsmenge an konzentriertem wäßrigem Ammoniak kurz erwärmt. Nach dem Abdunsten bzw. Abdestillieren des Ammoniak-Überschusses hinterbleibt das 1,2,4-Triazol-3-carbonsäureamid in quantitativer Ausbeute. Es ist in allen Eigenschaften (z. B. dem IR-Spektrum) identisch mit einer auf literaturbekanntem Weg, ausgehend vom entsprechenden Methylester, hergestellten Probe (z. B. Latvijas PSR Zinatnu Akad. Vestis, Khim. Ser. 1965, Seite 204, zitiert nach CA 63, 13 243 f [1965]).

**Patentansprüche**

1. Derivat des 2,5-Diketopiperazins der Formel

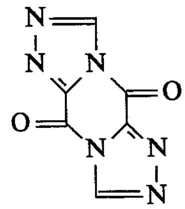

2. Verfahren zur Herstellung des Derivats des 2,5-Diketopiperazins nach Anspruch 1, dadurch gekennzeichnet, daß man die 1,2,4-Triazol-3-carbonsäure mit einer mindestens äquimolaren Menge eines Säurechlorids oder -bromids von Säuren des Schwefels, des Phosphors oder des Kohlenstoffes in Gegenwart von 0,5 bis 20 Mol-% Dimethylformamid, gegebenenfalls in Gegenwart eines inerten Lösungs- bzw. Verdünnungsmittels, bei erhöhter Temperatur umsetzt.

3. Verfahren nach Anspruch 2, dadurch gekennzeichnet, daß man als Säurechlorid Thionylchlorid einsetzt.

4. Verfahren nach Anspruch 2 und 3, dadurch gekennzeichnet, daß man 2 bis 10 Mol Thionylchlorid pro Mol 1,2,4-Triazol-3-carbonsäure einsetzt und das Thionylchlorid gleichzeitig als Lösungs- bzw. Verdünnungsmittel verwendet.

5. Verfahren nach Anspruch 1 bis 4, dadurch gekennzeichnet, daß man als erhöhte Temperatur die Siedetemperatur des Thionylchlorids einstellt.

6. Verwendung des 2,5-Diketopiperazinderivats nach Anspruch 1 zur Herstellung von 1,2,4-Triazolcarbonsäureamid.

**Claims**

1. Derivative of 2,5-diketopiperazine, of the formula

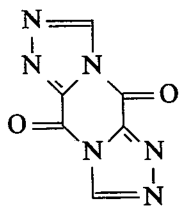

2. Process for the preparation of the derivative of 2,5-diketopiperazine according to Claim 1, characterised in that 1,2,4-triazole-3-carboxylic acid is reacted with at least an equimolar amount of an acid chloride or bromide of acids of sulphur, phosphorus or carbon, in the presence of 0.5 to 20 mol-% of dimethylformamide, if appropriate in the presence of an inert sulvent or diluent, at elevated temperature.

3. Process according to Claim 2, characterised in that thionyl chloride is used as the acid chloride.

4. Process according to Claim 2 and 3, characterised in that 2 to 10 mols of thionyl chloride are employed per mol of 1,2,4-triazole-3-carboxylic acid and the thionyl chloride is simultaneously used as the solvent or diluent.

5. Process according to Claim 1 to 4, characterised in that the elevated temperature established is the boiling point of thionyl chloride.

6. Use of the 2,5-diketopiperazine derivative according to Claim 1 for the preparation of 1,2,4-triazole-carboxylic acid amide.

**Revendications**

1. Dérivé de la 2,5-dicétopipérazine de formule

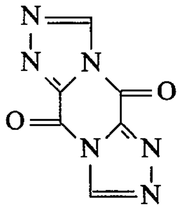

2. Procédé de production du dérivé de la 2,5-dicétopipérazine suivant la revendication 1, caractérisé en ce quón fait réagir à température élevée l'acide 1,2,4-triazole-3-carboxylique avec une quantité au moins équimolaire d'un chlorure ou d'un bromure d'acides du soufre, du phosphore ou du carbone en présence de 0,5 à 20 moles-% de diméthylformamide, éventuellement en présence d'un solvant ou d'un diluant inerte.

3. Procédé suivant la revendication 2, caractérisé en ce qu'on utilise comme chlorure d'acide le chlorure de thionyle.

4. Procédé suivant les revendications 2 et 3, caractérisé en ce qu'on utilise 2 à 10 moles de chlorure de thionyle par mole d'acide 1,2,4-triazole-3-carboxylique et on utilise le chlorure de thionyle en même temps comme solvant ou comme diluant.

5. Procédé suivant les revendications 1 à 4, caractérisé en ce qu'on choisit comme température élevée la température d'ébullition du chlorure de thionyle.

6. Utilisation du dérivé de 2,5-dicétopipérazine suivant la revendication 1 pour la production de l'amide d'acide 1,2,4-triazole-carboxylique.